# EUROPÄISCHE PATENTSCHRIFT

(11) **EP 2 401 060 B1**
(45) Veröffentlichungstag und Bekanntmachung des Hinweises auf die Patenterteilung: **25.11.2015**
(21) Anmeldenummer: 10707252.2
(22) Anmeldetag: 25.02.2010
(51) Int. Cl.: C07C 45/50, B01D 61/02, B01D 61/42, B01J 31/18, B01J 31/40

(54) **VERFAHREN ZUR ABTRENNUNG UND TEILWEISEN RÜCKFÜHRUNG VON RHODIUM BZW. DESSEN KATALYTISCH WIRKSAMEN KOMPLEXVERBINDUNGEN AUS PROZESSSTRÖMEN**
METHOD FOR SEPARATION AND PARTIAL RETURN OF RHODIUM AND CATALYTICALLY EFFECTIVE COMPLEX COMPOUNDS THEREOF FROM PROCESS STREAMS
PROCÉDÉ DE SÉPARATION ET DE RÉINTRODUCTION PARTIELLE DE RHODIUM OU DE SES COMPOSÉS COMPLEXÉS CATALYTIQUEMENT ACTIFS DANS DES FLUX DE PROCESSUS

(30) Priorität: 27.02.2009 DE 102009001230
(43) Veröffentlichungstag der Anmeldung: 04.01.2012
(73) Patentinhaber: Evonik Degussa GmbH, 45128 Essen (DE)
(72) Erfinder: PRISKE, Markus, 45768 Marl (DE); BAUMGARTEN, Götz, 45721 Haltern am See (DE); KAIZIK, Alfred, 45772 Marl (DE); LÜKEN, Hans-Gerd, 45770 Marl (DE)
(86) Internationale Anmeldenummer: PCT/EP2010/052390
(87) Internationale Veröffentlichungsnummer: WO 2010/097428

(56) Entgegenhaltungen:
- EP-A1- 0 895 811
- EP-A1- 1 103 303
- WO-A1-01/72679
- DE-A1- 2 311 388
- DE-A1- 10 308 111
- DE-A1- 19 801 437
- DE-A1-102005 046 250
- US-A- 3 978 148
- US-A- 5 215 667

## Beschreibung

Die vorliegende Erfindung betrifft die Abtrennung bzw. Anreicherung von Übergangsmetallen bzw. deren katalytisch wirksamen Komplexverbindungen aus Prozessströmen durch eine Kombination aus Membrantrennschritt und Adsorptionsschritt.

Insbesondere bei der rhodiumkatalysierten Hydroformylierung ist die Rückgewinnung des wertvollen Rhodiums aus den Produkt- als auch aus den Ausschleuseströmen ein wichtiger Prozessschritt, der zu einer Verbesserung der Wertschöpfung beiträgt.

Eine Vielzahl von Druckschriften befassen sich mit Rhodiumrückgewinnung. Die Verfahren beruhen auf Extraktion (EP0147824, WO01056932), Ionenaustausch (DE 195 43 15, US 5,208,194, WO 02/096848, WO 02/020451), Adsorption (US 3,539,634) oder Filtration (FR 1588014).

Die Art des vorliegenden Rhodiums beeinflusst dessen Adsorbierbarkeit auf dem Adsorbens. Einfluss hierauf hat die Temperatur sowie die Konzentration an Synthesegas (M. Beller, "Catalytic Carbonylation Reactions" in: Topics in Organometallic Chemistry, 18, (2006)).

DE 95 36 05 beschreibt die Abscheidung von gelöstem Rhodium durch Druckentspannung und Erhitzen des Produktstroms sowie die Adsorption mit Kieselgel oder Aktivkohle. Erzielbare Abscheideraten und Beladbarkeiten werden nicht genannt. Eine Erhitzung kann zu unerwünschter Aldolkondensation des Produktes führen.

In GB 801 734 und FR 1 588 014 wird eine metallische Ausfällung des Rhodiums durch Erhitzen mit Wasser erreicht. Die Rhodiumausfällungen werden anschließend mittels Dekanter, Filtration oder Zentrifuge vom Produkt entfernt. Die Zuführung von Wasser führt neben der Notwendigkeit eines weiteren thermischen Produktaufarbeitungsschritts dazu, dass der Katalysator nicht mehr der Reaktion zurückgeführt werden kann, da schon Spuren an Wasser die aktive Katalysatorspezies zerstören können.

Wird die Adsorption von Rhodium unter Wasserstoffatmosphäre durchgeführt, so lässt sich nach DE 22 62 852 das adsorbierte Rhodium in lösliche Rhodiumcarbonylkomplexe überführen. Als Adsorptionsmittel werden beispielhaft Aktivkohle, Kieselgel, Aluminiumoxid, Kieselgur und Magnesiumoxid aufgeführt. In den Beispielen wird der Einfluss der Wasserstoffatmosphäre während der Adsorption nicht gezeigt. Zudem handelt es sich bei der Beispielreaktion um ein wässriges System, welches z. B. auf ligandmodifizierte Hydroformylierungsreaktionen nicht übertragbar ist.

DE 23 11 388 beschreibt die selektive Adsorption von Rhodium-tert.-Phosphin-Katalysatoren an Oxide und Carbonate der Elemente der II. und III. Hauptgruppe des Periodensytems sowie der Silikate und Erdalkalimetalle. Das Verfahren ist sehr aufwendig, da der Katalysator mit speziellen Lösemitteln vom Absorbens gespült werden muss und anschließend von diesem Lösemittel getrennt werden muss. Diese zusätzlichen Schritte können sich schädigend auf den Katalysator auswirken. Zudem sind die Beladbarkeiten des Adsorbens sehr gering, was insgesamt zu einem nicht wirtschaftlichen Verfahren führt.

In US 4,388,279 wird die Extraktion von Rhodium mittels einer konzentrierten wässrigen Ammoniaklösung mit anschließender Adsorption an Calciumsulfat beschrieben. Das Verfahren ist sehr aufwendig und die Abscheideraten sind mit 71 bis 75 % sehr gering. Zudem wird keine Aussage über die Beladbarkeit des Adsorbens gemacht.

Der Einsatz von Aktivkohle zur adsorptiven Rhodiumabtrennung aus einem Prozess zur Herstellung von 1,4-Hexadien aus Ethylen und 1,3-Butadien bei Temperaturen von -20 °C bis +120 °C ist in US 3,978,148 offenbart. Die dabei erreichten Abscheideraten sind eher unbefriedigend. Im besten Beispiel beträgt die Rhodium-Konzentration im Ablauf immerhin noch 27 ppm. Zudem findet bei den eingesetzten Aktivkohlebetten ein sofortiger Durchbruch des Rhodiums mit äußerst raschem Anstieg der Rhodiumkonzentration im Eluat statt, was wiederum auf eine sehr geringe Beladbarkeit der Aktivkohle zurückzuführen ist. Die Möglichkeit eines direkten Recyclings in die Reaktion besteht nicht.

WO 01/72679 beschreibt ein Verfahren zur Rückgewinnung von Rhodium aus Hydroformylierungsprodukten. Gekennzeichnet ist das Verfahren dadurch, dass man die Hydroformylierungsprodukte in Gegenwart eines festen Adsorbens auf Temperaturen von 50 bis 200 °C erhitzt. Die Beladbarkeit der verwendeten Aktivkohle als entscheidender Faktor für die Wirtschaftlichkeit des Verfahrens wird nicht dargelegt. Ebenso nachteilig ist an dem Verfahren, dass eine direkte Rückführung des abgetrennten Rhodiums in die Reaktion nicht möglich ist.

Die Rückhaltung von Rhodium aus Hydroformylierungsgemischen mittels Adsorption ist dem Fachmann auch aus US3978148A1 bekannt.

EP 1 669 337 beschreibt ein Verfahren zur Adsorption von Cobalt. Die Abscheiderate von Cobalt auf Aktivkohle ist mit weniger als 9 % äußerst gering.

Weitere Schriften beschreiben die Rückgewinnung von Rhodium durch Ausfällung an Tellur oder Schwefel (DE 32 23 501, DE 29 11 193). Diese Verfahren sind aufwendig und kostenintensiv und nur schwer praktisch umsetzbar. Das ausgefällte Rhodium muss zudem aufwendig aufgearbeitet werden bevor es als aktiver Katalysator der Reaktion zugeführt werden kann.

Der Nachteil eines allein auf einem Adsorptionsschritt beruhenden Verfahrens, insbesondere zur Rückgewinnung von Rhodium, liegt im vollständigen Verlust der katalytischen Aktivität des gebundenen Metalls. Für den erneuten Einsatz als Hydroformylierungskatalysator ist eine aufwendige und kostenintensive metallurgische Aufarbeitung erforderlich. Hinzu kommt eine erhöhte Kapitalbindung.

DE10308111A1 offenbart ein Verfahren zur Abtrennung und teilweise Rückführung von Übergangsmetallen und/oder deren Katalytisch aktiven Komplexverbindungen aus einem Reaktionsgemisch mittels einer mindestens einstufigen Membranabtrennung und mit einer Rückführung des übergangsmetallangereicherten Retentatstrom zum Reaktionsgemisch.

Der Nachteil einer allein auf Nanofiltration beruhenden Membrantrennung liegt in der Tatsache, daß die Abtrennung prinzipiell nicht vollständig sein kann. Begegnet man diesem Nachteil durch Einsatz mehrstufige Verfahren, so steigen die Invest- und Betriebskosten mit der Stufenzahl bezüglich der zurückgehaltenen Rhodiummenge sehr stark an.

Die technische Aufgabe der Erfindung ist es, ein Verfahren zur Anreicherung von übergangsmetallbasierten Homogenkatalysatoren bereitzustellen, bei dem das Katalysatorsystem unter Erhalt seiner Aktivität angereichert bzw. abgetrennt werden kann. Das erfindungsgemäße Verfahren weist einen hohen Rückhaltegrad für das Katalysatorsystem auf und vermeidet möglichst dessen Zersetzung.

Diese technische Aufgabe wird gelöst durch ein Verfahren nach Anspruch 1. Vorteilhaft ist die erfindungsgemäße Kombination eines Membrantrennschritts per Nanofiltration mit einem Adsorptionsschritt. Mit der Nanofiltration wird ein Großteil des Rhodiums der Produkt- und Ausschleuseströme in dem Prozess gehalten. Für die Rückgewinnung des restlichen Rhodiums aus dem Permeatstrom der Nanofiltration eignet sich besonders die Adsorption auf veraschbaren Adsorbern.

Übergangsmetalle, insbesondere Rhodium, aus dem Permeatstrom einer Nanofiltration können:
i) an Liganden gebunden,
ii) teilweise an Liganden gebunden, oder
iii) frei in organischer Phase vorliegen.

Verfahrensbedingt ist der Permeatstrom einer Nanofiltration dadurch gekennzeichnet, dass er feststofffrei ist. Es befindet sich somit nur gelöstes und kein partikuläres Übergangsmetall, insbesondere Rhodium, im Permeatstrom.

Wie dem Fachmann bekannt ist, verbessert sich das Verhältnis von Adsorption zu Desorption bei niedrigeren Temperaturen. Dieser Effekt kann mit dem erfindungsgemäßen Verfahren vorteilhaft durch Erhitzen in einem Zwischenschritt zwischen Nanofiltration und Adsorption ausgenutzt werden, da die Adsorption von Übergangsmetallen, insbesondere von Rhodium, der thermischen Behandlung nachgeschaltet ist.

In erfindungsgemäßer Ausführung des Verfahrens zur Abtrennung und teilweisen Rückführung von Übergangsmetallen oder deren katalytisch wirksamen Komplexverbindungen wird aus einem Reaktionsgemisch, welches Hochsieder und ein Katalysatorsystem enthält, mittels eines Membrantrennschritts, welcher eine oder mehrere Membranen enthält, ein- oder mehrstufig verschaltet ist, die Metallkomponente zumindest zu 60 Massen-% im Retentatstrom zurückgehalten. Der Permeatstrom dieses Membrantrennschritts wird in dem nachfolgenden Adsoptionsschritt aufgearbeitet, wobei die zugeführte Metallkomponente zumindest zu 60 Massen-% von dem Adsorptionsmittel zurückgehalten wird. Hochsieder im Sinne der Erfindung sind Stoffe, die höher als die primären Hydroformylierungsprodukte (Aldehyde und/oder Alkohole mit einem C-Atom mehr als das eingesetzte Olefin) sieden und höhere Molmassen aufweisen und während der Hydroformylierung entstehen. Dazu gehören Aldolisierungsprodukte und Acetalisierungsprodukte, Ether sowie Ester, die durch Reaktion von Alkoholen und Säuren entstehen, wobei die Alkohole und Säuren durch Disproportionierung von Aldehyden gebildet werden. Hochsieder, die in Prozessströmen aus der Hydroformylierung vorhanden sind, besitzen im allgemeinen Siedepunkte über 55 °C bei 0,1 MPa.

Die Trennung im Membrantrennschritt wird mit einer Trenngrenze von 200 bis 2000 g/mol im Temperaturbereich von 40 bis 150 °C und in einem Bereich des transmembranen Druckes von 0,5 bis 7,0 MPa durchgeführt. Der Permeatstrom des Membrantrennschritts wird einem Adsorptionsschritt zugeführt und die Adsorption wird bei einer Temperatur von 30 bis 140 °C und Raumbelastungen von 0,01 bis 5 1/h durchgeführt. Bevorzugt wird das Adsorptionsmittel als Festbett eingesetzt.

Als Adsorptionsmittel können insbesondere Aktivkohleoberflächenreiche Polykieselsäuren wie Silicagele (Kiesel-Xerogele), hochdisperse Kieselsäure, oberflächenreiche Aluminiumoxide und Alumiumoxidhydrate als auch gebrauchte oder neue (Hydrier-)Katalysatoren eingesetzt werden.

Als besonders vorteilhafte Adsorbentien haben sich chemisch modifizierte Silica-Materialien erwiesen, wie sie in WO 2006013060 A1 offenbart sind. Derartige Adsorptionsmittel sind unter der Artikelbezeichnung Mercaptoalkyl-modified Silica, Type Rh H3, Batch No. 09-S26-001 der Firma PhosphonicS Ltd, 114 Milton Park, Abingdon, OXON, OX14 4SA, United Kingdom erhältlich.

Der Membrantrennschritt kann unter Einsatz von einer Membrane, zwei oder mehren Membranen oder unter Einsatz von einem, zwei oder mehreren Membrantrennschritt(en) durchgeführt werden. In dem erfindungsgemäßen Verfahren können insbesondere zwei oder mehrere Membrantrennschritte durchgeführt werden. Die Membrantrennschritte können direkt hintereinander durchgeführt werden. Die Hintereinanderschaltung kann in der Weise erfolgen, dass entweder der Retentatstrom oder der Permeatstrom, vorzugsweise der Permeatstrom des ersten Membrantrennschritts als Zuführstrom in einen weiteren Membrantrennschritt geleitet wird. Die auf den ersten erfindungsgemäßen Membrantrennschritt gegebenenfalls folgenden Membrantrennschritte können ebenfalls unter ähnlichen Bedingungen wie der erste durchgeführt werden. In einem Membrantrennschritt können eine Membran oder mehrere Membranen eingesetzt werden. Vorzugsweise werden in einem Membrantrennschritt zwei oder mehrere Membranen eingesetzt.

Bei einem mehrstufigen Membrantrennverfahren kann es vorteilhaft sein in den Membrantrennschritten Membranen mit unterschiedlichen Trenngrenzen und/oder Permeabilitäten einzusetzen.

In dem erfindungsgemäßen Verfahren werden Membranen eingesetzt, die auf Grund ihrer chemischen und/oder physikalischen Eigenschaften geeignet sind, Metall-Komplexkatalysator und/oder freien Organophosphor-Ligand vorzugsweise in einem Maße von zumindest 60 Massen-% zurückzuhalten. Eine weitere Voraussetzung für die Verwendbarkeit der Membrane besteht darin, dass die Membrane stabil gegenüber allen in der Hydroformylierungs-Reaktionsmischung vorhandenen Verbindungen, insbesondere gegenüber den Lösemitteln sein muss.

Vorzugsweise werden Membranen eingesetzt, die eine trennaktive Schicht aus einem Material, ausgewählt aus Celluloseacetat, Cellulosetriacetat, Cellulosenitrat, regenerierter Cellulose, Polyimiden, Polyamiden, Polyetheretherketonen, aromatischen Polyamiden, Polyamidimiden, Polybenzimidazolen, Polybenzimidazolone, Polyacrylnitril, Polyarylethersulfone, Polyester, Polycarbonaten, Polytetrafluorethylen, Polyvinylidenfluorid, Polypropylen, Polydimethylsiloxan (PDMS), Polyetheretherketon (PEEK), Acrylonitril/Glycidylmethacrylat (PANGMA), mit Silanen hydrophobisierte keramische Membranen, wie sie in DE 103 08 111 beschrieben sind, Polymere mit intrinsischer Mikroporösität (PIM) und anderen, wie sie z. B. in EP 0 781 166 und in "Membranes" von I. Cabasso, Encyclopedia of Polymer Sience and Technlogy, John Wiley and Sons, New York, 1987, beschrieben werden, aufweisen.

Besonders bevorzugt werden Membranen eingesetzt, die als trennaktive Schicht eine Polymerschicht aus Polydimethylsiloxan (PDMS), Polyimid (PI), Polyamidimid (PAI), Acrylonitril/Glycidylmethacrylat (PANGMA), Polyamid (PA) oder Polyetheretherketon (PEEK) aufweisen, die aus Polymeren mit intrinsischer Mikroporosität (PIM) aufgebaut sind, oder wobei die trennaktive Schicht über eine hydrophobierte keramische Membran aufgebaut ist. Ganz besonders bevorzugt werden Membranen aus Silikonen oder Polyamidimid eingesetzt. Solche Membranen sind kommerziell erhältlich. Neben den oben genannten Materialien können die Membranen weitere Materialien aufweisen. Insbesondere können die Membranen Stütz- oder Trägermaterialien aufweisen, auf die die trennaktive Schicht aufgebracht ist. Bei solchen Verbundmembranen liegt neben der eigentlichen Membrane noch ein Stützmaterial vor. Eine Auswahl von Stützmaterialien beschreibt EP 0 781 166, auf welches explizit verwiesen wird. Weiterhin können in der erfindungsgemäß einsetzbaren Membrane Verstärkungsmaterialien vorhanden sein, wie z. B. Partikel von anorganischen Oxiden oder anorganische Fasern, wie z. B. Keramik- oder Glasfasern, die die Stabilität der Membrane, insbesondere gegenüber Druckschwankungen oder hohen Druckdifferenzen, erhöhen.

Der Adsorptionsschritt kann in einer oder mehreren Batchvorlagen oder bevorzugt in einem oder mehreren Festbetten durchgeführt werden. Dabei kann bei Verwendung mehrerer Batchvorlagen und/oder Festbetten eine parallele oder serielle Verschaltung erfolgen.

Die Trennleistung des Adsorptionsschritts kann durch Rückführung des Eluats vor den Adsorptionsschritt erhöht werden.

Vorteilhaft ist die Verwendung von Aktivkohle als Adsorptionsmittel. Zum einen ist Aktivkohle kostengünstig und in großen Mengen verfügbar. Zum anderen kann die Rückgewinnung des adsorbierten Metalls durch Veraschen der beladenen Aktivkohle erfolgen. Beispiele für die Verwendung findende Aktivkohle, die kommerziell erhältlich ist, zeigt die Tabelle 1:

**Tabelle 1**

| **Lieferant** | **Handelsname** | **Melassezahl [mg]** | **Jodzahl, min. [mg/g]** | **BET [m²/g]** |
|---|---|---|---|---|
| Norit | GAC 1240 W | | 950 | 1100 |
| Norit | Gran | 150 | | 1400 |
| Chemviron | CPG® LF | 210 | 950 | 950 |
| Chemviron | AquaCarb | | 1100 | 1100 |
| Chemviron | CAL® I | 250 | 1050 | 1050 |
| Fluka | 18002 | | 1040 | |

Die Melassezahl sowie die Jodzahl sind allgemein akzeptierte analytische Parameter zur Beschreibung des Adsorptionsverhaltens von Aktivkohle. Weitere Details findet man z. B. in EP 1 280 593 B1 oder DE 34 18 150 A1.

Alternativ kann als Adsorbens ein chemisch modifiziertes Silica-Material verwendet werden, welches unter der Artikelbezeichnung Mercaptoalkyl-modified Silica, Type Rh H3, Batch No. 09-S26-001 von der der Firma PhosphonicS Ltd, 114 Milton Park, Abingdon, OXON, OX14 4SA, United Kingdom erhältlich ist. Näher beschrieben ist dieses Material in WO 2006013060 A1. Auf den Offenbarungsgehalt dieser Druckschrift wird insoweit Bezug genommen.

Je nach katalytisch wirksamer Komplexverbindung kann es vorteilhaft sein den an Übergangsmetall verarmten Strom des Membrantrennschritts in einem Zwischenbehälter verweilen zu lassen, um das Übergangsmetall oder dessen katalytisch wirksame Komplexverbindungen in gut adsorbierbare Spezies zu überführen. Gegebenenfalls vorteilhaft kann sich die weitere Zugabe an Liganden auf die Adsorbierbarkeit auswirken.

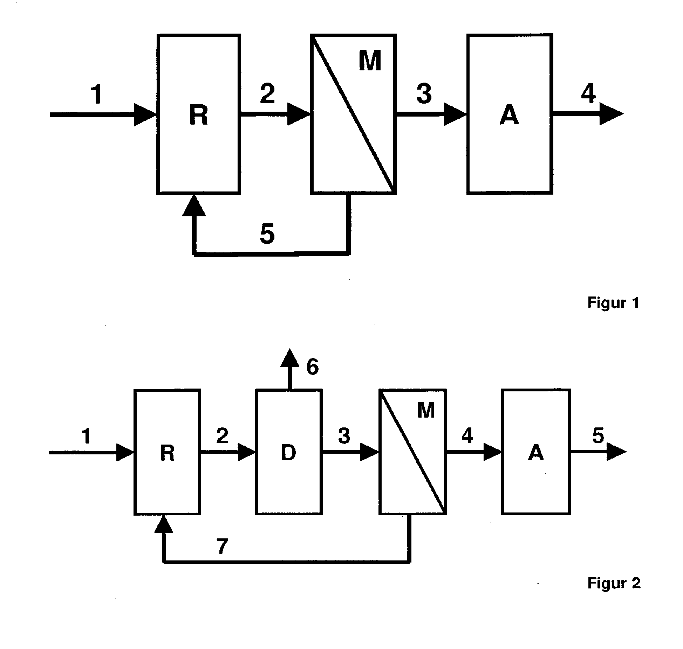

Nach Figur 1 werden die Edukte der Hydroformylierung, Olefine und Synthesegas (1), dem Reaktor (R) zugeführt. In Gegenwart der im Reaktor vorgelegten katalytisch wirksamen Komplexverbindungen findet die Hydroformylierung der Olefine zu Aldehyden statt. Aldehyde, wie auch Neben- und Folgeprodukte, darunter Hochsieder, wie z.B. Aldolkondensationsprodukte, nicht umgesetzte Edukte sowie die katalytisch wirksamen Komplexverbindungen, werden als Reaktionsgemisch aus dem Reaktor abgeführt (2) und einem Membrantrennschritt (M) zugeführt. Dabei findet retentatseitig (5) eine Anreicherung und permeatseitig (3) eine Abreicherung des Übergangsmetalls oder der katalytisch wirksamen Komplexverbindungen statt. Der an Übergangsmetall oder katalytisch wirksamen Komplexverbindungen abgereicherte Strom (3) wird dem Adsoptionsschritt (A) zugeführt aus dem der weiter an Übergangsmetall oder katalytisch wirksamen Komplexverbindungen abgereicherte Strom (4) ausströmt.

Sollte eine direkte Abtrennung der Hochsieder von dem Hydroformylierungsprodukt Aldehyd notwendig sein, so zeigt Figur 2 eine bevorzugte Prozessverschaltung. Die Edukte der Hydroformylierung, Olefine und Synthesegas (1), werden dem Reaktor (R) zugeführt. In Gegenwart der im Reaktor vorgelegten katalytisch wirksamen Komplexverbindungen findet die Hydroformylierung der Olefine zu Aldehyden statt. Aldehyde, wie auch Neben- und Folgeprodukte, darunter Hochsieder, wie z. B. Aldolkondensationsprodukte, nicht umgesetztes Edukt sowie das Übergangsmetall oder dessen katalytisch wirksame Komplexverbindungen, werden als Reaktionsgemisch aus dem Reaktor abgeführt (2) und einem thermischen Trennschritt (D) zugeführt. In dem thermischen Trennschritt wird das Reaktionsgemisch (2) in einen Strom an höher siedenden Komponenten, wie die zuvor beschriebenen Nebenprodukte der Reaktion sowie mit Übergangsmetall und/oder dessen katalytisch wirksamen Komplexverbindungen angereicherten Strom (3) und in einen an leichter siedenden, überwiegend Aldehyde enthaltenden Produktstrom (6) getrennt. Der Hochsiederstrom (3) wird einem selektiven Membrantrennschritt (M) zugeführt. Dabei findet retentatseitig (7) eine Anreicherung und permeatseitig (4) eine Abreicherung des Übergangsmetalls oder dessen katalytisch wirksamen Komplexverbindungen statt. Der an Übergangsmetall oder katalytisch wirksamen Komplexverbindungen abgereicherte Strom (4) wird dem Adsoptionsschritt (A) zugeführt, aus dem der weiter an Übergangsmetall oder katalytisch wirksamen Komplexverbindungen abgereicherte Strom (5) ausströmt. Das erfindungsgemäße Verfahren zur Abtrennung und teilweisen Rückführung von Rhodium und dessen katalytisch wirksamen Komplexverbindungen aus Reaktionsgemischen von Hydroformylierungsprozessen durch eine Kombination aus Membrantrennschritt und Adsorptionsschritt wird im Folgenden beispielhaft für die Rhodiumkomplex-katalysierte Hydroformylierung von C12-haltigen Olefingemischen beschrieben.

Dieser Gegenstand der Erfindung ist die Anwendung des erfindungsgemäßen Verfahrens bei der Herstellung von Tridecanalen und Tridecanole-haltigen Gemischen, wie z. B. in EP 1 515 934 B1 dargestellt, wobei die Abtrennung des Rhodium-haltigen Katalysatorkomplexes folgende Schritte umfasst:
a) thermische Abtrennung des Tridecanale- und Tridecanole-haltigen Produktgemisches aus dem Reaktionsgemisch der Hydroformylierung;
b) mindestens einstufige Membrantrennung, die an mindestens einer Membrane erfolgt, so dass mindestens 60 Massen-% des eingesetzten Rhodiums im Retentatstrom verbleiben;
c) Zuführung des erhaltenen Permeatstroms zu einem nachfolgenden Adsorptionsschritt, so dass mindestens 60 Massen-% des im Permeatstrom enthaltenen Rhodium zurückgehalten werden;
d) Rückführung des Retentatstroms mit dem angereicherten Rhodium-haltigen Katalysatorkomplex aus Schritt b) in die Hydroformylierungsreaktion.

Es ist vorteilhaft, wenn der Membrantrennschritt des erfindungsgemäßen Verfahrens bei einer transmembranen Druckdifferenz von mehr als 1,5 MPa erfolgt. Eine hohe transmembrane Druckdifferenz führt zum einen zu einem erhöhten Rückhalt des Übergangsmetalls bzw. dessen katalytisch wirksamen Komplexverbindungen und zum anderen zu einem erhöhten Permeatstrom je eingesetzter Membranfläche.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen. Nachfolgend wird die vorliegende Erfindung anhand von Beispielen näher erläutert. Alternative Ausführungsformen der vorliegenden Erfindung sind in analoger Weise erhältlich.

### Beispiele

### Erfindungsgemäßes Beispiel Nr.1

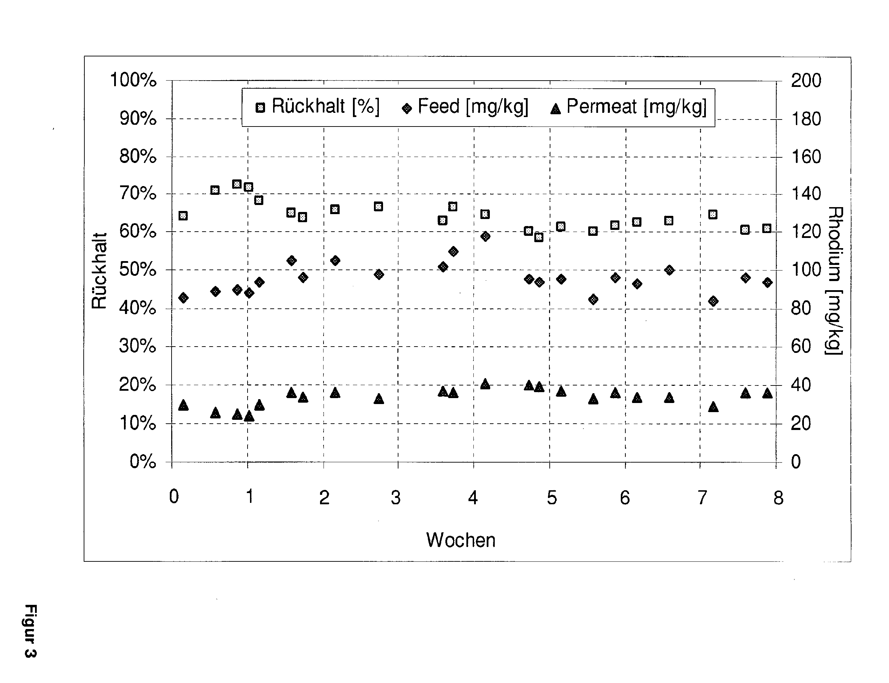

Als Beispiel wurde die Trennaufgabe gemäß Figur 2 gewählt. Bei der Reaktion handelt es sich um eine Rhodium-Phosphit-katalysierte Hydrofomylierung eines C12-haltigen Olefingemisches, wie z. B. in EP 1 515 934 B1 dargestellt. Die verwendete Rhodiumvorstufe ist Rhodiumacetylacetonatodicarbonyl und der eingesetzte Ligand ist Tris(2,4-di-tert-butyl-phenyl)phosphit. Die Rhodiumkonzentration betrug 10 mg/kg bezogen auf das Reaktionsgemisch. Das Ligand zu Rhodiumverhältnis betrug 20:1. Das Aldehydgemisch aus Tridecanalen, wie auch Neben- und Folgeprodukte, darunter Hochsieder, wie z. B. Aldolkondensationsprodukte, nicht umgesetztes Edukt sowie Rhodium oder die katalytisch wirksamen Komplexverbindungen werden einem thermischen Trennschritt zugeführt. Der an Hochsieder und Rhodium oder die katalytisch wirksamen Komplexverbindungen enthaltende (ca. 100 ppm) angereicherte Sumpf wird dem kombinierten Membrantrenn- und Adsorptionsschritt zugeführt. Der Hochsiederanteil liegt bei über 50 Massen-%. Die Molmassendifferenz zwischen aktiver Katalysatorspezies und den Hochsiedern liegt unter 500 g/mol. Beispielhaft konnte gezeigt werden, dass mit einem einstufigen Membrantrennschitt mit einer strahlenchemisch modifizierten Silikonkompositmembran (Lieferant Fa. GMT, Typ oNF2), wie sie z.B. in DE 195 07 584 C2 beschrieben ist, bei einer Temperatur von 80°C, einem Transmembrandruck von 2,0 MPa und einer Überströmung der Membran von 1,7 m/s über 60 Massen-% des Katalysators aus dem Hochsiederstrom zurückgehalten werden (Figur 3).

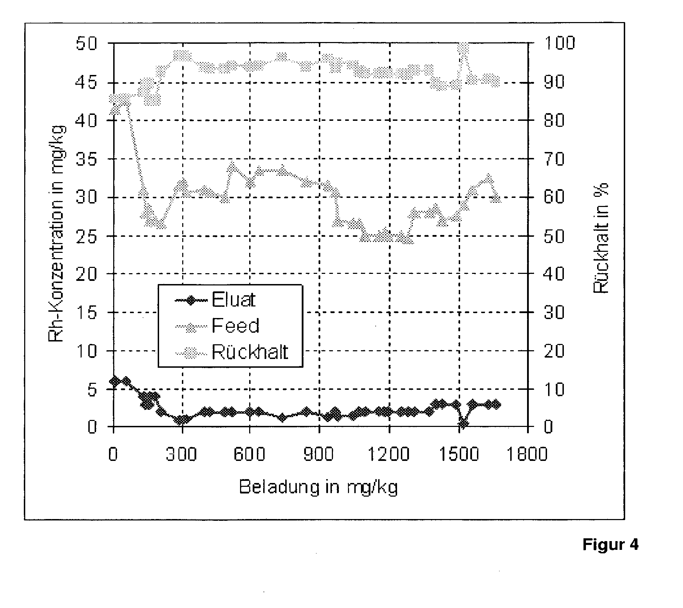

Die im Permeatstrom verbleibende Katalysatorfracht wurde mit einer Raumbelastung von ca. 0,1 m³/(m³h) über ein 1 m hohes Aktivkohlebett aus 614 g gekörnter Aktivkohle (Lieferant Fa. Fluka Aktivkohle gekörnt, purum, Artikel 18002, Charge 52980) geleitet, wobei weitere 85 bis 99 Massen-% des Katalysators zurückgehalten wurden. In den Versuchen wurden Beladungen der Aktivkohle mit Rhodium von mehr als 1,5 mg/kg erreicht (siehe Figur 4).

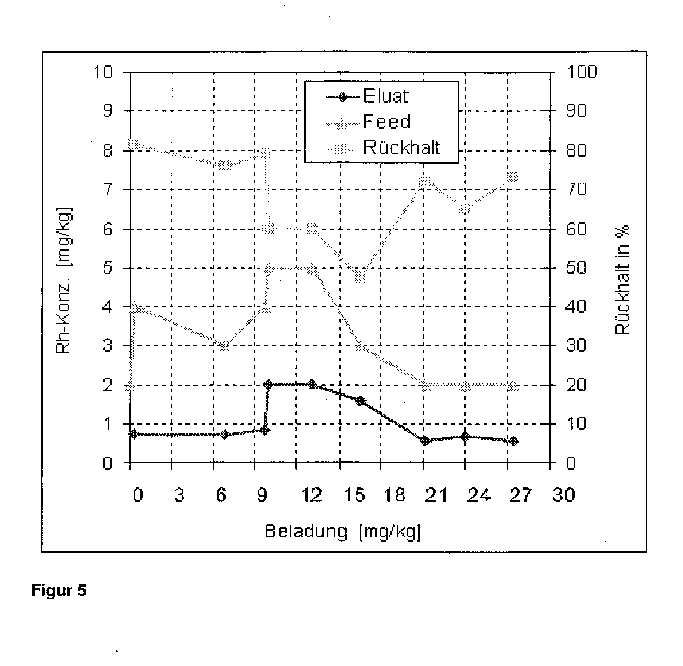

Wird das so erhaltene, an Rhodium abgereicherte Eluat mit einer Raumbelastung von ca. 0,1 m³/(m³h) über ein 1 m hohes Aktivkohlebett aus weiteren 614g gekörnter Aktivkohle (gleiche Qualität) geleitet, so wird die Rhodiumfracht um weitere 50 bis 80 % reduziert (siehe Figur 5).

### Erfindungsgemäßes Beispiel Nr. 2

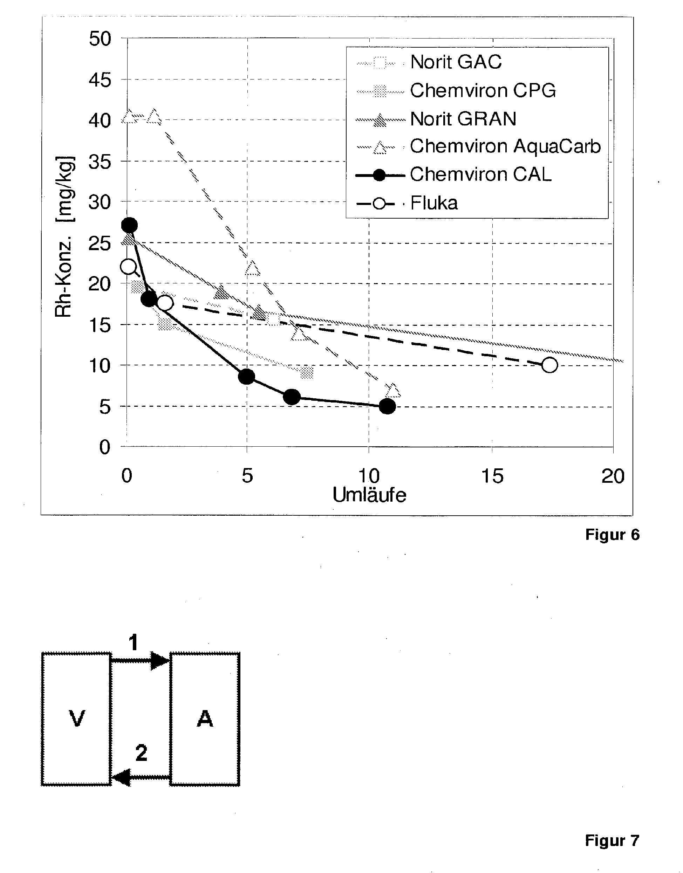

In diesem Beispiel wurden einige weitere, kommerziell erhältliche Aktivkohletypen (s. Tabelle 1) mit einem analog zum vorherigen Beispiel gewonnen Permeatstrom aus einer Batchvorlage (V) gemäß Figur 7 mit dem Strom 1 beaufschlagt. Eingesetzt wurden jeweils 60 g Aktivkohle bei einer Raumbelastung von 1 m³/(m³h). Das gewonnene Eluat, Strom 2, wurde dabei mehrere Mal über das Aktivkohlebett A rezirkuliert, wobei die Rhodiumkonzentration im Eluat mit steigender Zahl der Umläufe deutlich abnimmt (Figur 6).

Bei einer gegebenen Aktivkohleschüttung lassen sich für einen gegebenen Zulaufstrom niedrige Rhodiumkonzentrationen im Eluat bevorzugt durch eine Batchfahrweise mit Rezirkulation des Eluat erreichen.

### Erfindungsgemäßes Beispiel Nr. 3

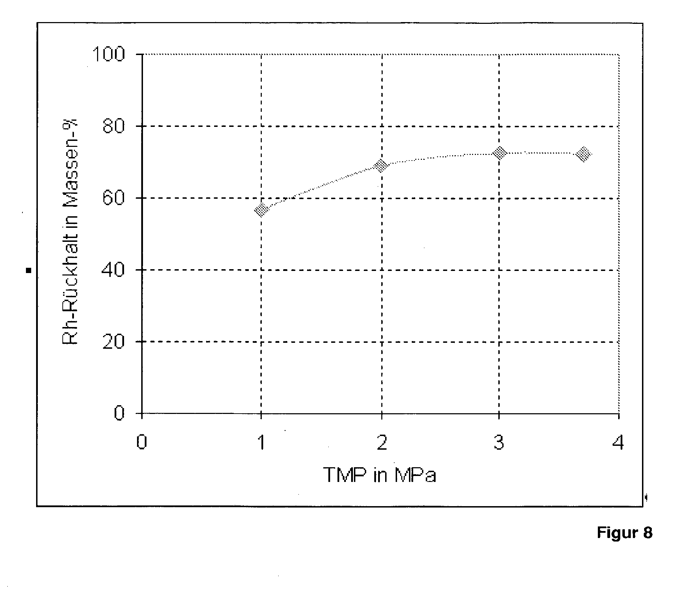

Der analog zum Beispiel Nr. 1 gewonnene Sumpfstrom wurde in einem Membrantrennschritt bei verschiedenen Transmembrandrücken (1 MPa, 2 MPa, 3 MPa, 3,7 MPa) über eine strahlenchemisch modifizierte Silikonkompositmembran (Lieferant Fa. GMT, Typ oNF2) geführt. Dabei ergaben sich nach Figur 8 deutliche bessere Rhodiumrückhalte bei höheren Transmembrandrücken.

### Erfindungsgemäßes Beispiel Nr. 4

In einem weiteren erfindungsgemäßen Beispiel wurde der gemäß Beispiel 1 erzeugte Permeatstrom:
im Fall A direkt und
im Fall B mit vorgeschalteter Erhitzung für 24 Stunden bei 90 °C über eine Aktivkohleschüttung von 60 g mit einer Raumbelastung von 1 m³/(m³h) und einer Schüttungstemperatur von 100 °C im einfachen Durchlauf geführt.

Der 40 mg/kg Rhodium enthaltene Permeatstrom wurde im Fall A auf 14 mg/kg und im Fall B auf 6 mg/kg abgereichert.

### Erfindungsgemäßes Beispiel Nr. 5

Ein gemäß Beispiel 1 erhaltenes Hochsieder- und Rhodium-Komplexkatalysatorangereichertes Sumpfprodukt wurde in eine analog zu Beispiel 1 betriebene zweistufigen Membranfiltration gefahren. Dabei wurde ein Rhodium-Rückhalt von rund 90 % erzielt. Der nunmehr abgereicherte Permeatstrom wurde bei einer Temperatur von 70°C mit einer Raumbelastung von 0,5 m³/(m³/h) auf die Schüttung eines chemisch modifizierten Silica-Materials (Mercaptoalkyl-modified Silica, Type Rh H3, Batch No. 09-S26-001 der Fa. PhosphonicS) gefahren. Die Rhodium-Konzentration im Eluat lag unter 0.5 ppm. Die entspricht insgesamt einem Rhodium-Rückhalt von mehr als 99 %.

## Patentansprüche

1. Verfahren zur Abtrennung und teilweisen Rückführung von Übergangsmetallen und/oder deren katalytisch wirksamen Komplexverbindungen aus einem Reaktionsgemisch (2) durch Kombination einer mindestens einstufigen Membrantrennung (M) und einer Adsorption (A), wobei ein katalysatorhaltiger Strom (2,3), enthaltend ein Übergangsmetall, über mindestens einen einstufigen Membrantrennschritt (M) in einen übergangsmetallangereicherten Retentatstrom (5, 7), welcher dem Reaktionsgemisch (2) wieder zugeführt, und einen übergangsmetallabgereicherten Permeatstrom (3, 4) aufgeteilt wird und weiter der übergangsmetallabgereicherte Permeatstrom (3, 4) einem Adsorptionsschritt (A) zugeführt wird,
wobei das Übergangsmetall Rhodium ist,
wobei mindestens 60 Massen-% Rhodium mittels des Membrantrennschrittes (A) im Retentatstrom (5, 7) verbleiben,
und wobei mindestens 60 Massen-% Rhodium des Permeatstroms (3, 4) nach dem Membrantrennschritt (M) mittels Adsorption (A) abgetrennt werden.

2. Verfahren nach Anspruch 1,
**dadurch gekennzeichnet,**
**dass** der Membrantrennschritt (M) mit einer Trenngrenze im Bereich von 200 bis 2000 g/mol durchgeführt wird.

3. Verfahren nach den vorhergehenden Ansprüchen,
**dadurch gekennzeichnet, dass** der Membrantrennschritt (M) in einem Temperaturbereich von 40 bis 150 °C durchgeführt wird.

4. Verfahren nach den vorhergehenden Ansprüchen,
**dadurch gekennzeichnet,**
**dass** der Membrantrennschritt (M) in einem Bereich des transmembranen Druckes von 0,5 bis 7 MPa durchgeführt wird.

5. Verfahren nach den vorhergehenden Ansprüchen,
**dadurch gekennzeichnet,**
**dass** der Adsorptionsschritt (A) an Aktivkohle durchgeführt wird.

6. Verfahren nach mindestens einem der Ansprüche 1 bis 4,
**dadurch gekennzeichnet,**
**dass** als Adsorbens ein chemisch modifiziertes Silica-Material verwendet wird.

7. Verfahren nach Anspruch 5 oder 6,
**dadurch gekennzeichnet,**
**dass** das Adsorbens als Schüttung überströmt wird.

8. Verfahren nach Anspruch 7,
**dadurch gekennzeichnet,**
**dass** das Eluat des Adsorptionsschrittes (A) auf das Adsorbens zurückgeführt wird.

9. Verfahren nach den vorhergehenden Ansprüchen,
**dadurch gekennzeichnet,**
**dass** der Adsorptionsschritt (A) in einem Temperaturbereich von 30 bis 140 °C durchgeführt wird.

10. Verfahren nach den vorhergehenden Ansprüchen,
**dadurch gekennzeichnet,**
**dass** der Adsorptionsschritt (A) mit einer Raumbelastung im Bereich von 0,01 bis 5 h⁻¹ durchgeführt wird.

11. Verfahren nach den vorhergehenden Ansprüchen,
**dadurch gekennzeichnet,**
**dass** der Adsorptionsschritt (A) in einem Festbett durchgeführt wird.

12. Verfahren nach den vorhergehenden Ansprüchen zur Herstellung von Tridecanalen und Tridecanole-haltigen Gemischen (6), wobei die Abtrennung des Rhodium-haltigen Katalysatorkomplexes folgende Schritte umfasst:
a) thermische Abtrennung des Tridecanale- und Tridecanole-haltigen Produktgemisches (6) aus dem Reaktionsgemisch (2) der Hydroformylierung (R);
b) mindestens einstufige Membrantrennung (M), die an mindestens einer Membrane erfolgt, so dass mindestens 60 Massen-% des eingesetzten Rhodiums im Retentatstrom (7) verbleiben;
c) Zuführung des erhaltenen Permeatstroms (4) zu einem nachfolgenden Adsorptionsschritt (A), so dass mindestens 60 Massen-% des im Permeatstrom (4) enthaltenen Rhodiums zurückgehalten werden;
d) Rückführung des Retentatstroms (7) mit dem angereicherten Rhodium-haltigen Katalysatorkomplex aus Schritt b) in die Hydroformylierungsreaktion (R).

## Claims

1. Process for separating off and partly recirculating transition metals and/or catalytically active complexes thereof from a reaction mixture (2) by a combination of an at least single-stage membrane separation (M) and an adsorption (A), wherein a catalyst-containing stream (2,3) containing a transition metal is separated by means of at least one single-stage membrane separation step (M) into a retentate stream (5,7) enriched in transition metal, which is returned to the reaction mixture (2), and a permeate stream (3,4) depleted in transition metal and the permeate stream (3,4) depleted in transition metal is fed to an adsorption step (A),
wherein, the transition metal is rhodium,
wherein at least 60% by mass of rhodium remains in the retentate stream (5,7) in the membrane separation step (M),
and wherein at least 60% by mass of rhodium in the permeate stream (3,4) after the membrane separation step (M) is separated off by means of adsorption (A).

2. Process according to Claim 1, **characterized in that** the membrane separation step (M) is carried out with a separation limit in the range from 200 to 2000 g/mol.

3. Process according to any of the preceding claims, **characterized in that** the membrane separation step (M) is carried out in a temperature range from 40 to 150°C.

4. Process according to any of the preceding claims, **characterized in that** the membrane separation step (M) is carried out in a transmembrane pressure range from 0.5 to 7 MPa.

5. Process according to any of the preceding claims, **characterized in that** the adsorption step (A) is carried out over activated carbon.

6. Process according to at least one of Claims 1 to 4, **characterized in that** a chemically modified silica material is used as adsorbent.

7. Process according to Claim 5 or 6, **characterized in that** the liquid flows over the adsorbent as a bed.

8. Process according to Claim 7, **characterized in that** the eluate from the adsorption step (A) is recirculated to the adsorbent.

9. Process according to any of the preceding claims, **characterized in that** the adsorption step (A) is carried out in a temperature range from 30 to 140°C.

10. Process according to any of the preceding claims, **characterized in that** the adsorption step (A) is carried out at a space velocity in the range from 0.01 to 5 h⁻¹.

11. Process according to any of the preceding claims, **characterized in that** the adsorption step (A) is carried out in a fixed bed.

12. Process according to any of the preceding claims for preparing mixtures (6) containing tridecanals and tridecanols, wherein the removal of the rhodium-containing catalyst complex comprises the following steps:
a) thermal separation of the product mixture (6) containing tridecanals and tridecanols from the reaction mixture (2) from the hydroformylation (R) ;
b) at least single-stage membrane separation (M) which is carried out over at least one membrane so that at least 60% by mass of the rhodium used remains in the retentate stream (7);
c) introduction of the permeate stream (4) obtained into a subsequent adsorption step (A) so that at least 60% by mass of the rhodium present in the permeate stream (4) is retained;
d) recirculation of the retentate stream (7) with the concentrated rhodium-containing catalyst complex from step b) to the hydroformylation reaction (R).

## Revendications

1. Procédé de séparation et de recyclage partiel de métaux de transition et/ou de leurs composés complexes catalytiquement actifs à partir d'un mélange réactionnel (2) par combinaison d'une séparation sur membrane à au moins une étape (M) et d'une adsorption (A), un courant contenant le catalyseur (2, 3), contenant un métal de transition, étant partagé par une étape de séparation sur membrane à au moins une étape (M) en un courant de rétentat enrichi en métal de transition (5, 7), qui est réintroduit dans le mélange réactionnel (2), et un courant de perméat appauvri en métal de transition (3, 4), et le courant de perméat appauvri en métal de transition (3, 4) étant introduit dans une étape d'adsorption (A),
le métal de transition étant le rhodium,
au moins 60 % en masse du rhodium restant dans le courant de rétentat (5, 7) au moyen de l'étape de séparation sur membrane (M),
et au moins 60 % en masse du rhodium du courant de perméat (3, 4) étant séparé après l'étape de séparation sur membrane (M) par l'adsorption (A).

2. Procédé selon la revendication 1, **caractérisé en ce que** l'étape de séparation sur membrane (M) est réalisée avec une limite de séparation dans la plage allant de 200 à 2 000 g/mol.

3. Procédé selon les revendications précédentes, **caractérisé en ce que** l'étape de séparation sur membrane (M) est réalisée dans une plage de température allant de 40 à 150 °C.

4. Procédé selon les revendications précédentes, **caractérisé en ce que** l'étape de séparation sur membrane (M) est réalisée dans une plage de pression transmembranaire de 0,5 à 7 MPa.

5. Procédé selon les revendications précédentes, **caractérisé en ce que** l'étape d'adsorption (A) est réalisée sur du charbon actif.

6. Procédé selon au moins l'une quelconque des revendications 1 à 4, **caractérisé en ce qu'**un matériau de silice modifié chimiquement est utilisé en tant qu'adsorbant.

7. Procédé selon la revendication 5 ou 6, **caractérisé en ce que** l'adsorbant est inondé en tant que garnissage.

8. Procédé selon la revendication 7, **caractérisé en ce que** l'éluat de l'étape d'adsorption (A) est recyclé sur l'adsorbant.

9. Procédé selon les revendications précédentes, **caractérisé en ce que** l'étape d'adsorption (A) est réalisée dans une plage de température allant de 30 à 140 °C.

10. Procédé selon les revendications précédentes, **caractérisé en ce que** l'étape d'adsorption (A) est réalisée avec un chargement spatial dans la plage allant de 0,01 à 5 h⁻¹.

11. Procédé selon les revendications précédentes, **caractérisé en ce que** l'étape d'adsorption (A) est réalisée dans un lit fixe.

12. Procédé selon les revendications précédentes pour la fabrication de mélanges contenant des tridécanals et des tridécanols (6), la séparation du complexe catalytique contenant du rhodium comprenant les étapes suivantes :
a) la séparation thermique des mélanges de produits contenant des tridécanals et des tridécanols (6) à partir du mélange réactionnel (2) de l'hydroformylation (R) ;
b) la séparation sur membrane à au moins une étape (M), qui a lieu sur au moins une membrane, de manière à ce qu'au moins 60 % en masse du rhodium utilisé reste dans le courant de rétentat (7) ;
c) l'introduction du courant de perméat obtenu (4) dans une étape d'adsorption (A) ultérieure, de manière à ce qu'au moins 60 % en masse du rhodium contenu dans le courant de perméat (4) soit récupéré ;
d) le recyclage du courant de rétentat (7) contenant le complexe catalytique contenant du rhodium enrichi de l'étape b) dans la réaction d'hydroformylation (R).
